# EUROPEAN PATENT SPECIFICATION

(11) **EP 2 376 103 B1**
(45) Date of publication and mention of the grant of the patent: **12.12.2012**
(21) Application number: 10700089.5
(22) Date of filing: 07.01.2010
(51) Int. Cl.: A61K 38/13, A61K 9/107, A61K 8/64, A61Q 3/02, A61Q 3/00, A61P 17/06

(54) **CYCLOSPORINE COMPOSITIONS FOR ENHANCING NAIL GROWTH**
CYCLOSPORINZUSAMMENSETZUNGEN FÜR ERHÖHTES NAGELWACHSTUM
COMPOSITIONS DE CYCLOSPORINE POUR RENFORCER LA CROISSANCE DES ONGLES

(30) Priority: 08.01.2009 US 143317 P; 17.03.2009 US 160946 P; 26.05.2009 US 181165 P; 27.05.2009 US 181353 P
(43) Date of publication of application: 19.10.2011
(73) Proprietor: Allergan, Inc., Irvine, CA 92612 (US)
(72) Inventor: MULLINS, Debbie, Bruceville Texas 76630 (US); STUCKER, Connie, Lott Texas 76656 (US); WHITCUP, Scott M., Laguna Hill California 92653 (US); SCHIFFMAN, Rhett M., Laguna Beach California 92651 (US); WALT, John G., Huntington Beach California 92648 (US)
(74) Representative: HOFFMANN EITLE
(86) International application number: PCT/US2010/020376
(87) International publication number: WO 2010/080913

(56) References cited:
- EP-A1- 0 547 229
- WO-A1-95/31211
- DE-A1- 19 529 085
- US-A1- 2005 059 583
- CANNAVÒ S P ET AL: "Treatment of psoriatic nails with topical cyclosporin: a prospective, randomized placebo-controlled study." DERMATOLOGY (BASEL, SWITZERLAND) 2003 LNKD- PUBMED:12592084, vol. 206, no. 2, 2003, pages 153-156, XP9134400 ISSN: 1018-8665
- SYUTO TOMOKO ET AL: "Successful treatment of psoriatic nails with low-dose cyclosporine administration." EUROPEAN JOURNAL OF DERMATOLOGY : EJD 2007 MAY-JUN LNKD- PUBMED:17478392, vol. 17, no. 3, May 2007 (2007-05), pages 248-249, XP002585865 ISSN: 1167-1122
- JIARAVUTHISAN ET AL: "Psoriasis of the nail: Anatomy, pathology, clinical presentation, and a review of the literature on therapy" JOURNAL OF THE AMERICAN ACADEMY OF DERMATOLOGY, C.V. MOSBY, ST. LOUIS, MO, US LNKD- DOI:10.1016/J.JAAD.2005.07.073, vol. 57, no. 1, 1 July 2007 (2007-07-01), pages 1-27, XP022111711 ISSN: 0190-9622
- PIERARD G E ET AL: "Dynamics of psoriatic trachyonychia during low-dose cyclosporin A treatment: A pilot study on onychochronobiology using optical profilometry" DERMATOLOGY (BASEL), vol. 192, no. 2, 1996, pages 116-119, XP9134407 ISSN: 1018-8665

## Description

### Field of the Invention

The present application is directed to cyclosporine compositions for enhancing nail and cuticle growth.

### Background of the Invention

Nails, including toenails and fingernails, are primarily composed of the protein keratin. Fingernails and toenails are living tissue. The nail is formed in a pocket of skin which has grown inward and is called the nail matrix. This area generates the nail and is also called the root of the nail. The outer layer of the matrix contains specialized cells that create the keratin that grows out as the nail plate.

The nail plate is the commonly referred to as a fingernail or toenail.

The nail bed is the finger tissue or toe tissue that supports the nail. Nails grow continuously throughout a person's life, growing approximately at an average rate of 3 millimeters a month. Fingernails may require 3 to 6 months to re-grow completely, and toenails require approximately 12 to 18 months. Actual growth rate is dependent upon age, gender, season, exercise level, diet, and hereditary factors.

The treatment of fingernail and toe nail problems due to various conditions such as anemia, vitamin or mineral deficiency, onycohrrhexis, kidney and liver disorders, psoriasis, vascular disease (e.g., Raynaud's disease) and heart disease often result in poor nail growth, vertical trenches, pitting, cracking, horizontal lines, lack of thickness and strength, lack of smoothness and tendency to tear remains a significant problem. Despite the development of numerous treatments such as the use of nail enhancement and hardening polishes, protein elixirs such as bovine collagen emulsions, various types of wearable protective fingernail covers and other similar products, there remains a significant need for treatments which actually accelerate the growth of nails and cuticles and restore the health of nails and cuticles. The problem with current treatments for improving nail health is that they are mainly cosmetic in nature, largely ineffective and time consuming. There is a need for compositions which actually enhance or accelerate the growth of nails and cuticles and restore nails and cuticles to their natural health to thicken, strengthen and smooth the nail.

### Summary of the Invention

The present invention is directed to cyclosporine formulations and methods for stimulating the growth of nails and cuticles (eponychium) in a mammal, including humans and includes both finger nails and toe nails. The compositions may be administered topically to the nail bed, nail matrix, the end or tip of the nail and cuticle in an amount effective to increase nail growth and thicken, strengthen and smooth the nail and cuticle and to treat a nail disorder. The composition may be a pharmaceutical composition or a composition for cosmetic use.

The compositions of the present invention are comprised of cyclosporine topical compositions which may be in the form of solutions, emulsions, reverse or micro emulsions, colloids, suspensions, dispersions, gels, pastes and polishes containing cyclosporine, in particular cyclosporine A, cyclosporine A derivatives and components, and may also include cyclosporine B - Z, the structures of which are well known in the art. The composition of the present invention may be administered concurrently with other therapeutics such as orally administered anti-fungal agents.

The compositions of the present invention are also useful for treating conditions and diseases of the nail such as nail psoriasis, psoriatic nail dystrophy, onychia, onychiagryposis, onychia trophia, onychocryptosis, onychodystrophy, onychogryposis, onycholysis, onychomadesis, onychauxis, onychomycosis, onychorrhexis, tinea unguium, onychophosis, onychoptosis, paronychia, pseudomonas, pterygium and pterygium inversum unguis, koilonychia, subungual hematoma or other trauma to the nail, folic acid deficiency, leukonychia, nail patella syndrome, melanonychia, protein deficiency, brittle and peeling nails, methyl methacrylate damaged nails, vitamin C deficiency, vitamin deficiency, tinea unguis, thinning nails associated with lichen planus, Raynaud's disease, beeding associated with rheumatoid arthritis, beau's lines, and Mee's lines associated with certain kinds of poisoning.

Also part of the present disclosure is the following:
1. A composition for use in accelerating toenail or finger nail growth, treating brittle toe or finger nails in a human or animal, the composition comprising:
   a composition selected from the group consisting of an emulsion, solution, gel, paste, dispersion or suspension comprising cyclosporine.
2. The formulation of paragraph 1 wherein the cyclosporine is cyclosporine A.
3. The formulation of paragraphs 1 - 2 wherein the cyclosporine is a cyclosporine A derivative or segment.
4. The formulation of paragraphs 1 - 3 wherein the composition is an emulsion.
5. The formulation of paragraphs 1- 4 wherein the cyclosporine is cyclosporine A and is present in an emulsion in the amount of 0.05% w/v.
6. The formulation of paragraphs 4 and 5 wherein the emulsion also contains glycerin, castor oil, polysorbate 80 and carbomer 1342.
7. The formulation of paragraphs 2 and 4 - 5 where in the cyclosporine is cyclosporine A and is present in a solution in the amount 0.01 - 0.0125 % w/v.
8. The formulation of paragraph 1 wherein the cyclosporine is present in the amount of 0.001 - 50% w/v of the composition.
9. A method of accelerating nail growth, in a human or animal, the method comprising:
   applying a composition selected from the group consisting of an emulsion, solution, gel, paste, dispersion or suspension comprising cyclosporine,
   wherein the emulsion, solution, gel, paste, dispersion or suspension comprising cyclosporine is applied topically to the nails, cuticles or nail matrix of the human or animal at least once a day.
10. The method of paragraph 9 wherein the cyclosporine is cyclosporine A.
11. The method of paragraph 9 wherein the cyclosporine is a cyclosporine A derivative or segment.
12. The method of paragraph 10 wherein the composition is an emulsion.
13. The method of paragraph 12 wherein the cyclosporine is cyclosporine A and is present in an emulsion in the amount of 0.05% w/v.
14. The method of paragraphs 9 - 13 wherein the emulsion also contains glycerin, castor oil, polysorbate 80 and carbomer 1342.
15. The method of paragraphs 9 - 13 wherein the cyclosporine is cyclosporine A and is present in a solution in the amount 0.01 - 0.0125 % w/v.
16. The method of paragraph 9 wherein the cyclosporine is present in the amount of 0.001 - 50% w/v of the composition.
17. A composition for use in treating a disorder selected from the group consisting of nail psoriasis, psoriatic nail dystrophy, onychia, onychiagryposis, onychia trophia, onychocryptosis, onychodystrophy, onychogryposis, onycholysis, onychomadesis, onychauxis, onychomycosis, onychorrhexis, tinea unguium, onychophosis, onychoptosis, paronychia, pseudomonas, pterygium and pterygium inversum unguis, koilonychia, subungual hematoma or other trauma to the nail, folic acid deficiency, leukonychia, nail patella syndrome, melanonychia, protein deficiency, brittle and peeling nails, methyl methacrylate damaged nails, vitamin C deficiency, vitamin deficiency, tinea unguis, thinning nails associated with lichen planus, Raynaud's disease, beeding associated with rheumatoid arthritis, beau's lines, and Mee's lines wherein the composition is selected from the group consisting of an emulsion, solution, gel, paste, dispersion or suspension comprising cyclosporine.
18. The composition of paragraph 17 wherein the composition is applied from one of the following selected from the group consisting of once a day, twice a day, three times a day, four times a day, once a week, twice a week, three times a week, four times a week, five times a week or six times a week.
19. The composition of paragraphs 17 - 18 wherein the composition is applied with an applicator.
20. The composition of paragraphs 17 and 18 wherein the composition is contained in a kit comprising a vial or dispenser containing the composition and an applicator brush.
21. The composition of paragraphs 17 - 20 wherein cyclosporine is present in a concentration selected from the group consisting of 0.1 to 20 % w/v, 0.1 - 10 % w/v, 0.1 - 5% w/v, 0.1 - 1.0% w/v, 0.09% - 0.1% w/v, 0.08% - 0.1% w/v, 0.07% - 0.1% w/v, 0.06% - 0.1 % w/v, 0.05% - 0.1% w/v, 0.04% - 0.1 % w/v, 0.03% - 0.1% w/v, 0.02% - 0.1% w/v, 0.01% - 0.1% w/v, 0.01 - 0.09%, 0.01 - 0.08% 0.01 - 0.07% w/v, 0.01 - 0.06 % w/v, 0.01 - 0.05% w/v, 0.01 - 0.04% w/v, 0.01 - 0.03% w/v, 0.01 - 0.02% w/v, 0.01 - 0.0125% w/v and most preferably 0.01 to 0.05% or 0.01 % w/v, 0.0125% w/v, 0.02 % w/v, 0.03% w/v, 0.04 % w/v, 0.05% w/v, 0.06 % w/v, 0.07 % w/v, 0.08 % w/v, 0.09 % w/v, 0.1% w/v, 0.005% w/v, and 0.0015% w/v.
22. The composition of paragraphs 17 - 21 wherein the cyclosporine is selected from the group consisting of cyclosporine A, cyclosporine A derivatives, and cyclosporine A - Z and salts thereof.
23. The composition of paragraphs 17 - 22 for use in application to the cuticles.
24. The composition of paragraph 23 wherein application to the cuticles causes increased cuticle growth.
25. Use of the pharmaceutical or cosmetic composition containing cyclosporine for the manufacture of a medicament for the treatment of the following diseases selected from the group consisting of nail psoriasis, psoriatic nail dystrophy, onychia, onychiagryposis, onychia trophia, onychocryptosis, onychodystrophy, onychogryposis, onycholysis, onychomadesis, onychauxis, onychomycosis, onychorrhexis, tinea unguium, onychophosis, onychoptosis, paronychia, pseudomonas, pterygium and pterygium inversum unguis, koilonychia, subungual hematoma or other trauma to the nail, folic acid deficiency, leukonychia, nail patella syndrome, melanonychia, protein deficiency, brittle and peeling nails, methyl methacrylate damaged nails, vitamin C deficiency, vitamin deficiency, tinea unguis, thinning nails associated with lichen planus, Raynaud's disease, beeding associated with rheumatoid arthritis, beau's lines, and Mee's lines wherein the composition is selected from the group consisting of an emulsion, solution, gel, paste, dispersion or suspension.
26. The use of paragraph 25 wherein cyclosporine is present in a concentration selected from the group consisting of 0.1 to 20 % w/v, 0.1 - 10 % w/v, 0.1 - 5% w/v, 0.1 - 1.0% w/v, 0.09% - 0.1% w/v, 0.08% - 0.1% w/v, 0.07% - 0.1% w/v, 0.06% - 0.1% w/v, 0.05% - -0.1% w/v, 0.04% -0.1% w/v, 0.03% 0.1% w/v, 0.02% - 0.1% w/v, 0.01% - 0.1% w/v, 0.01 - 0.09%, 0.01 - 0.08% 0.01 - 0.07% w/v, 0.01 - 0.06 % w/v, 0.01 - 0.05% w/v, 0.01 - 0.04% w/v, 0.01 - 0.03% w/v, 0.01 - 0.02% w/v, 0.01 - 0.0125% w/v and most preferably 0.01 to 0.05% or 0.0 1 % w/v, 0.0125% w/v, 0.02 % w/v, 0.03% w/v, 0.04 % w/v, 0.05% w/v, 0.06 % w/v, 0.07 % w/v, 0.08 % w/v, 0.09 % w/v, 0.1 % w/v, 0.005% w/v and 0.0015% w/v.

### Detailed Description of the Invention

The cyclosporine compositions of the present invention are applied topically on the nail by an applicator, and can be administered multiple times a day. Concentrations of 0.01 to 50 % w/v of cyclosporine and particular cyclosporine A are hereby contemplated including 0.1 to 20 % w/v, 0.1 - 10 % w/v, 0.1 - 5% w/v, 0.1 - 1.0% w/v, 0.09% - 0.1% w/v, 0.08% - 0.1 % w/v, 0.07% - 0.1 % w/v, 0.06% - 0.1 % w/v, 0.05% - 0.1 % w/v, 0.04% - 0.1% w/v, 0.03% - 0.1% w/v, 0.02% - 0.1% w/v, 0.01% - 0.1% w/v, 0.01 - 0.09%, 0.01 - 0.08% 0.01 - 0.07% w/v, 0.01 - 0.06 % w/v, 0.01 - 0.05% w/v, 0.01 - 0.04% w/v, 0.01 - 0.03% w/v, 0.01 - 0.02% w/v, 0.01 - 0.0125% w/v, 0.001 - 0.009% w/v, 0.001 - 0.008% w/v, 0.001 - 0.007% w/v, 0.001 - 0.006% w/v, 0.001 - 0.005% w/v, 0.001 - 0.004% w/v, 0.001 - 0.003% w/v, 0.001 - 0.002% w/v, and 0.0001 - 0.001 % w/v and most preferably 0.01 to 0.05% or 0.01% w/v, 0.0125% w/v, 0.02 % w/v, 0.03% w/v, 0.04 % w/v, 0.05% w/v, 0.06 % w/v, 0.07 % w/v, 0.08 % w/v, 0.09 % w/v, 0.1% w/v, 0.005% w/v, 0.0015% w/v of native cyclosporine A, cyclosporine A and derivatives including cyclosporine B - Z.

In accordance with the present invention, cyclosporine A or cyclosporine A derivatives may be applied in any efficacious concentration, e.g., 0.01 to saturation (e.g. greater than 20 weight percent) in a pharmaceutically acceptable excipient.

Specific examples of pharmaceutically acceptable excipients may be olive oil, arachis oil, castor oil, mineral oil, petroleum jelly, dimethyl sulphoxide, chremophor, Miglyol 182 (commercially available from Dynamit Nobel Kay-Fries Chemical Company, Mont Vale, N.J.), an alcohol (e.g. ethanol, n-propyl alcohol, or iso-propyl alcohol), liposomes or liposome-like products or a silicone fluid. Preferred excipients are dimethyl sulphoxide and olive oil. Mixtures of at least two of any suitable excipients may be used.

The cyclosporine A derivatives are advantageously administered topically in drop form (solution, emulsion or suspension) or an ointment containing an effective amount of the derivative. Concentrations of 0.01 1 to 50 weight percent, preferably 0.1 to 20 weight percent, of the cyclosporine A derivatives are used in the practice of the present invention.

Cyclosporines are a group of nonpolar cyclic oligopeptides with known immunosuppressant activity. Cyclosporine A, along with several other minor metabolites, as well as cyclosporine B, C, D, E, F, G, H, I, J, K, L, M, N, O, P, Q, R, S, T, U, V, W, X, Y and Z, have been identified. In addition, derivatives, salts and the like of such cyclosporines and a number of synthetic analogs have been prepared and may be useful in the present invention. The use of cyclosporine-A and cyclosporine A derivatives to treat ophthalmic conditions has been the subject of various patents, for example Ding et al U.S. Patent 5,474,979; Garst U.S. Patent Nos. 6,254,860; 6,350,442, and 7,368,436.

In general, commercially available cyclosporines may contain a mixture of several individual cyclosporines which all share a cyclic peptide structure consisting of eleven amino acid residues with a total molecular weight of about 1,200, but with different substituents or configurations of some of the amino acids. Thus the present invention contemplates mixtures of different types of cyclosporines or cyclosporine components.

The term "cyclosporine component" as used herein is intended to include any individual member of the cyclosporine group, salts thereof, derivatives thereof, analogs thereof and mixtures thereof, as well as mixtures of two or more individual cyclosporines salts thereof, derivatives thereof, analogs thereof and mixtures thereof.

Particularly preferred cyclosporine components include, without limitation, cyclosporine A, derivatives of cyclosporine A, salts of cyclosporine A and the like and mixtures thereof. Cyclosporine A is an especially useful cyclosporine component.

The chemical structure for cyclosporine A is represented by Formula 1: 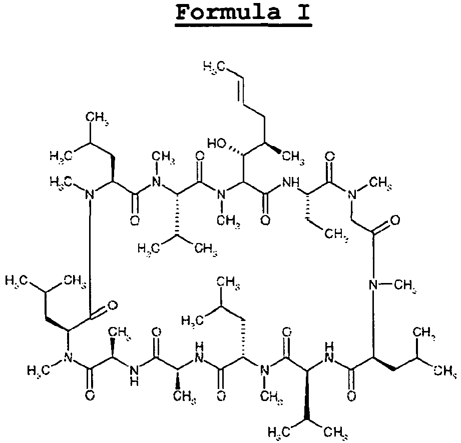 As used herein the term "derivatives" of a cyclosporine refer to compounds having structures sufficiently similar to the cyclosporine so as to function in a manner substantially similar to or substantially identical to the cyclosporine, for example, cyclosporine A, in the present methods. Included, without limitation, within the useful cyclosporine A derivatives are those selected from ((R)-methylthio-Sar)³-(4'-hydroxy-MeLeu) cyclosporine A, ((R)-(Cyclo)alkylthio-Sar)³-(4'-hydroxy-MeLeu)⁴-cyclosporine A, and ((R)-(Cyclo)alkylthio-Sar)³-cyclosporine A derivatives described below.

These cyclosporine derivatives are represented by the following general formulas (II), (III), and (IV) respectively: 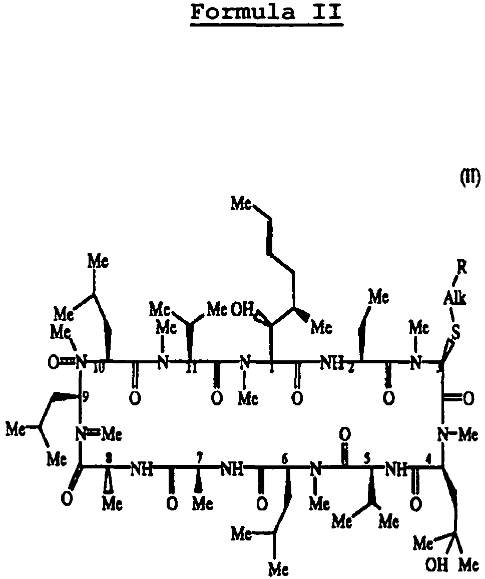 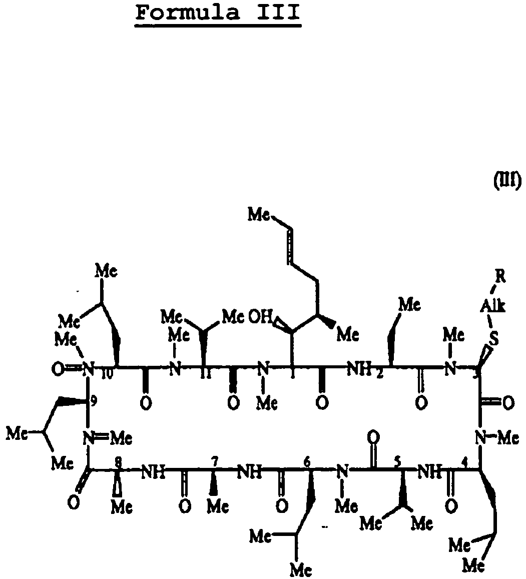 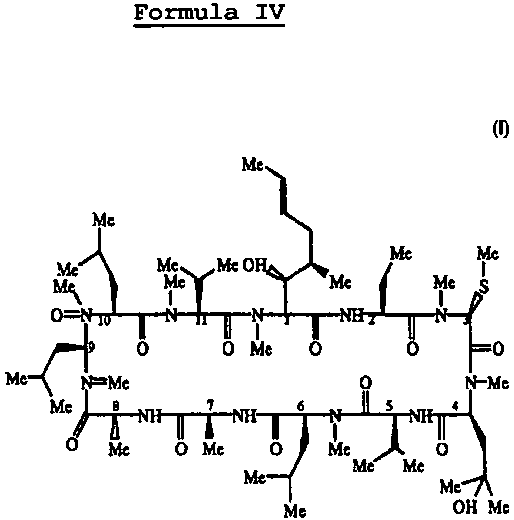 wherein Me is methyl; Alk is 2-6C alkylene or 3-6C cycloalkylene; R is OH, COOH, alkoxycarbonyl, -NR₁R₂ or N(R₃)-(CH₂)-NR₁R₂; wherein R₁,R₂ is H, alkyl, 3-6C cycloalkyl, phenyl (optionally substituted by halo, alkoxy, alkoxycarbonyl, amino, alkylamino or dialkylamino), benzyl or saturated or unsaturated heterocyclyl having 5 or 6 members and 1-3 heteroatoms; or NR₁R₂ is a 5 or 6 membered heterocycle which may contain a further N, O or S heteroatom and may be alkylated; R₃ is H or alkyl and n is 2-4; and the alkyl moieties contain 1-4C.

The present compositions and methods may be practiced employing any suitable compositions or combinations of compositions including therapeutically effective amounts of cyclosporine component useful for application to the nails including the tip or end of the nails, cuticles and nail matrix. The cyclosporine component is present in an amount effective to provide the desired therapeutic effect when the cyclosporine-containing composition is administered to a human or animal in accordance with the present invention. Mixtures of cyclosporine components are contemplated. In one embodiment of the invention, the cyclosporine component advantageously is present in the compositions in amounts ranging from about 0.01% to about 0.05% by weight or 15% or about 20% or more by weight of the composition. In another embodiment, the cyclosporine component is present in an amount of about 0.01% to about 5% or about 10% or about 15% by weight of the composition. Other concentrations of the cyclosporine component contemplated are 0.1 to 20 % w/v, 0.1 - 10 % w/v, 0.1 - 5% w/v, 0.1 - 1.0% w/v, 0.09% - 0.1% w/v, 0.08% - 0.1% w/v, 0.07% - 0.1% w/v, 0.06% - 0.1% w/v, 0.05% - 0.1% w/v, 0.04% - 0.1% w/v, 0.03% - 0.1% w/v, 0.02% - 0.1% w/v, 0.01% - 0.1% w/v, 0.01 - 0.09%, 0.01 - 0.08% 0.01 - 0.07% w/v, 0.01 - 0.06 % w/v, 0.01 - 0.05% w/v, 0.01 - 0.04% w/v, 0.01 - 0.03% w/v, 0.01 - 0.02% w/v, 0.01 - 0.0125% w/v and most preferably 0.01 to 0.05% or 0.01 % w/v, 0.0125% w/v, 0.02 % w/v, 0.03% w/v, 0.04 % w/v, 0.05% w/v, 0.06 % w/v, 0.07 % w/v, 0.08 % w/v, 0.09 % w/v or 0.1 % w/v of cyclosporine A and its derivatives. It is intended, however, that the choice of a particular amount of cyclosporine component is to be made in accordance with factors well known in the medicinal arts, including mode of administration, the size and condition of the human or animal and the type and severity of the condition to be treated.

Useful compositions for practicing the invention may be liquids, suspensions, emulsions, reverse emulsions, micro-emulsions, semi-solids, solutions, dispersions, capsules, gels, lotions, creams, pastes, polishes and the like. Those skilled in the art of pharmaceutical formulation are able to formulate suitable compositions including cyclosporine components in a suitable form, such as those forms noted herein, for example, including one or more pharmaceutically acceptable excipients, such as those conventionally used in similar compositions. Of course, care should be taken to use composition components which are compatible with the cyclosporine component being used and which do not unduly or significantly interfere with the administering step in which the composition is being used or with the human or animal being treated. One particular useful formulation of the present invention is an emulsion comprising 0.05 % w/v cyclosporine, 1.0% w/v polysorbate 80, 2.2% w/v glycerine, 1.25% w/v castor oil, 0.05% w/v pemulin, purified water and at a pH of 7.4.

For example, cyclosporine components can be combined with carriers which form emulsions upon mixing with water. Such emulsions are described, for example, and without limitation, in Cavanak U.S. Pat. No. 4,388,307. Carriers, for example, and without limitation, glyceride carriers, may assist in alleviating problems of physical instability such as precipitation of the cyclosporine component from solution, and may also enable higher blood plasma concentrations, if desired.

In a useful embodiment, the presently useful compositions include hydrophobic components. Any suitable hydrophobic component may be employed in the present invention. Advantageously, the cyclosporine component is solubilized in the hydrophobic component. In one embodiment, the hydrophobic component may be considered as comprising a discontinuous phase in the presently useful cyclosporine component-containing compositions, for example, oil-in-water emulsions.

The hydrophobic component may be present in an effective amount, for example, in an amount of up to about 1.0% by weight or about 1.5% by weight or more of the composition.

Preferably, the hydrophobic component comprises one or more oily materials. Examples of useful oil materials include, without limitation, vegetable oils, animal oils, mineral oils, synthetic oils and the like and mixtures thereof. In a very useful embodiment, the hydrophobic component comprises one or higher fatty acid glycerides. Excellent results are obtained when the hydrophobic component comprises castor oil.

Other useful cyclosporine component-containing compositions comprise the cyclosporine component in admixture with an emulsifying amount of a fatty acid glyceride, such as castor oil and the like, and a surfactant, such as polysorbate 80. Such compositions are described in Ding et al U.S. Patent 5,474,979, Also see Kaswan U.S. Patent 4,649,047 and Kaswan U.S. Patent 5,411,952.

In one embodiment, the presently useful compositions are self-emulsifying which, when exposed to an aqueous medium, form fine oil-in-water emulsions with little or no agitation. The property of self-emulsification permits such formulations to be administered in concentrated form, as for example in a soft gelatin or viscous paste. Additionally, emulsions may be prepared by combining a self-emulsifying pre-concentrate with an aqueous medium.

Previously-disclosed self-emulsifying systems include those in which a cycloporin component is combined with mixtures of (i) medium-chain triglycerides and nonionic surfactants, (ii) vegetable oils and partial glycerides, such as polyglycolized glycerides or medium-chain mono- and diglycerides, or (iii) vegetable oils and nonionic surfactants such as polysorbate 80 or PEG-25 glyceryl trioleate.

In certain self-emulsifying formulations, a "microemulsion preconcentrate" of a cyclosporine component is formed by combining the cyclosporine component with (I) a hydrophilic phase, (II) a lipophilic phase, and (III) a surfactant, as well as optional thickeners, antioxidants or other excipients. Examples of such compositions are disclosed in Hauer et al U.S. Patent 5,342,625.

In addition, suitable compositions may include cyclosporine components in combination with a hydrophilic solvent phase and one or more surfactants, but not containing lipophilic solvents. Such cyclosporine component-containing formulations may be stable, simple to prepare, and have good pharmacokinetic properties.

As used herein, the terms "binary system", "binary composition" and "binary system of excipients" denote those cyclosporine component-containing formulations and compositions which contain, in addition to the cyclosporine component, a combination of at least one hydrophilic solvent and at least one surfactant, but which lack a lipophilic solvent. Such compositions may be supplemented with additional adjuvants and still be considered "binary", so long as they do not include a lipophilic solvent phase.

To prepare such pharmaceutical compositions, a binary system is combined with a cyclosporine component. The hydrophilic phase may comprise one or more of the known pharmaceutically acceptable hydrophilic solvents or excipients that are capable of solubilizing the cyclosporine component. Suitable classes of hydrophilic compounds include, for example and without limitation, pharmaceutically acceptable alcohols including the polyethylene glycols.

Examples of hydrophilic phase components useful in the presently useful compositions include, but are not limited to, water, ethanol, benzyl alcohol, propylene glycol, low molecular weight polyethylene glycols having a molecular weight of up to about 1,000, glycol, dimethyl isosorbide and the like and mixtures thereof.

The hydrophilic phase, comprising one or more hydrophilic solvents, typically comprises about 10% to about 90% by weight of the pharmaceutical composition. The precise amount used will vary depending on the nature of the hydrophilic compound or compounds used, the amount of cyclosporine component present, the dosage form, the condition being treated and other factors known in the art. Preferably the hydrophilic phase comprises about 20% to about 80%, and more preferably about 30% to about 60%, by weight of the composition. Where non-aqueous hydrophilic components are used, water can be included in the formulation at levels varying from about 0.5% to about 10%, or preferably from about 1% to about 5%, based on total weight of the composition.

Any of the known pharmaceutically acceptable surfactants may be used, and mixtures of surfactants may be used, including nonionic, anionic, cationic, and combinations thereof. Nonionic surfactants are preferred, and especially those surfactants having a hydrophile/lipophile balance (HLB) of 10 or more. Alternatively, certain combinations of high- and low-HLB surfactants may be utilized; preferably, such mixed surfactants are used in ratio such that the aggregate surfactant HLB (when weighted according to proportions used) remains in excess of 10.

Examples of suitable surfactants include, but are not limited to, polyoxyethylene derivatives of natural or hydrogenated vegetable oils such as castor oil; polyoxyethylene-sorbitan fatty acid esters, such as mono-, di- and tri-lauryl, palmityl, stearyl and oleyl esters; alkyl/dialykyl sulfate, sulfonate or sulfosuccinate salts such as sodium lauryl sulfate and dioctyl sodium sulfosuccinate; polyoxyethylene fatty acid esters; phospholipids such as lecithins; transesterification products of natural vegetable oil triglycerides and polyalkylene polyols; sorbitan fatty acid esters; pentaerythritol fatty acid esters; polyoxyethylene glycol alkyl ethers and esters; and the like. The surfactants may be used alone or in combination.

Other examples of surfactants which may be used include, without limitation, polyoxyethylene castor oil derivatives, such as polyoxyethylene glycerol triricinoleate polyoxyl 35 castor oil (CREMOPHOR® EL, available from BASF Corporation), and polyoxyl 40 hydrogenated castor oil (CREMOPHOR® RH40, available from BASF Corporation); mono-fatty acid esters of poloxyethylene (20) sorbitan, such as polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), and polyoxyethylene (20) sorbitan monolaurate (TWEEN® 20) (all available from ICI Surfactants, Wilmington, Del.); polyoxyethylene glycol 200 monostearate (MYRJ® 52, available from Calgene Chemicals, Skokie, Ill.); polyglycerol esters with a HLB of 10 or greater, such as decablyceryl mono- and dioleate and the like; and mixtures thereof.

In some instances (as when the compositions are prepared as semi-solids), it may be advantageous to use at least one additional low-HLB surfactant along with one or more of the above high-HLB surfactant. Examples of low-HLB auxiliary surfactants which may be used include, but are not limited to, polyglycerol oleates (such as CAPROL® 10G40); lecithins; glyceryl monooleate or monolinoleate mixtures (such as MYVEROL® 18-99 or 18-92); propylene glycol laurate; and sorbitan oleates such as sorbitan monooleate (SPAN® 80), sorbitan trioleate (SPAN® 85), and sorbitan sesquioleate (SPAN® 20) (all available from ICI Surfactants, Wilmington, Del.). The surfactant phase may comprise about 10% to 90% by weight of the composition. Preferably the surfactant comprises about 20% to about 70% of the composition, and more preferably about 40% to about 60%, by weight.

If desired, the presently useful compositions may additionally comprise other pharmaceutically acceptable excipients, such as tonicity components, buffer components, polyelectrolyte components, thickeners, fillers, diluents, flavoring agents, coloring agents, antioxidants, preservatives, such as antibacterial or antifungal agents, acids and/or basis to adjust pH, and the like and mixtures thereof. Such additives, if present, may typically comprise about 0.01% to about 10% by weight of the composition. Such additives include those additives which are conventional and/or well known for use in similar pharmaceutical compositions. For example, suitable thickening agents include any of those known in the art, as for example pharmaceutically acceptable polymers and/or inorganic thickeners. Such agents include, but are not limited to, polyacrylate homo- and co- polymers; celluloses and cellulose derivatives; polyvinyl pyrrolidones; polyvinyl resins; silicates; and the like and mixtures thereof.

When desired, the cyclosporine-containing compositions may be prepared as semi-solid rather than liquid formulations by addition a greater proportion of appropriate thickening or solidifying agents. As noted above, such preparations may be particularly useful as fills for hard gelatin (as opposed to soft gelatin) capsules. Solidifiers suitable for the preparation of semi-solid compositions include, but are not limited to, polyethylene glycols having a molecular weight of more than about 1,000, such as PEG 1450 and PEG 3350; stearyl alcohol; and colloidal silicon dioxide (CAB-O-SIL® M-5, available from Cabot, Tuscola, Ill.). A semi-solid state may be often obtained by adding between about 8% or about 10% and about 15% or about 25% by weight solidifying agent. The actual amount of solidifying agent needed will depend on the physical characteristics of the other excipients which are present.

Except as otherwise noted elsewhere herein, the cyclosporine component-containing compositions are administered topically. However, other modes of administration are possible such as systemic administration, for example, oral administration of a capsule or suspension, intramuscular, intraperitoneal, subcutaneous and intraarticular injection or infusion of a cyclosporine component-containing composition. Topical administration includes ointments, drops, solutions, lacquers (pigmented nail polish or lacquer comprising a separate anti-fungal agent), suspensions or emulsions including a cyclosporine component which may be administered using an applicator system which are well known in the art. Topical formulations, intended for topical administration to the affected tissue area or areas, may be prepared directly, or by combining a cyclosporine component-containing concentrate with a diluent, for example, an aqueous diluent. Such topical formulations may include additional excipients as necessary, for example, to modify consistency of the rate of absorption of the cyclosporine component.

In preparing the presently useful compositions, the components may be combined in any order with mixing or light agitation to ensure complete blending.

The cyclosporine component may be administered in a sufficient amount, and for a sufficient time, as required to provide the desired therapeutic effect. The specific therapeutically effective dosage level may be dependent on a number of factors including the specific condition to be treated, the severity of the condition, the activity of the particular cyclosporine component being employed, the specific cyclosporine component-containing composition employed, the time and method of administration, the duration of treatment, and other factors which are well known in the medical arts.

The following non-limiting examples illustrate certain aspects of the present invention.

### Example 1

A female patient, age 51, with fingernails with pronounced horizontal ridges on her fingernails ("Beau's Lines") and poor nail growth, topically applies four times a day with an applicator brush an emulsion of 0.05% cyclosporine A sold under the tradename RESTASIS® by Allergan, Inc. of Irvine, California. After 7 days of application, the subject's fingernails will experience accelerated growth without the presence of horizontal ridges.

### Example 2

A male patient, age 44, with fingernails experiencing vertical splitting and pitting possibly due to vitamin or mineral deficiency, topically applies ten times a day with an applicator an emulsion of 0.05% cyclosporine A sold under the tradename RESTASISO® by Allergan, Inc. of Irvine, California. After ten days of application, the subject's fingernails will experience accelerated growth and increased thickness and the disappearance of pits at the portion of the nail growing upward from the eponychium and lunula area of the finger without the presence of pits.

### Example 3

A female patient, age 45, with brittle (onycohrrhexis) and thinning nails with poor growth, topically applies twelve times a day with a squeezable applicator, an aqueous solution of 0.01 % cyclosporine A. After ten days of application, the subject's fingernails will experience significant accelerated growth with thicker and less brittle nails emerging from the nail matrix.

### Example 4

A female patient, age 55, with fingernails with pronounced horizontal ridges ("Beau's Lines") and pits on her fingernails with poor nail growth, topically applies to her nails and cuticles twice a day with an applicator brush an emulsion of 0.05% cyclosporine A sold under the tradename RESTASIS® by Allergan, Inc. of Irvine, California. The 0.05% cyclosporine A emulsion is compounded in a lacquer for application to the nails and cuticles. After 7 days of application, the subject's fingernails will experience accelerated growth without the presence of horizontal ridges.

### Example 5

A male patient, age 55, with brittle fingernails suffering from onycohrrhexis with and poor nail growth, topically applies to his nails and cuticles twelve times a day with an applicator brush an emulsion of 0.05% cyclosporine A sold under the tradename RESTASIS® by Allergan, Inc. of Irvine, California. After 5 days of application, the subject's fingernails will experience accelerated growth without the presence of horizontal ridges.

### Example 6

A male patient, age 65, suffering from nail psoriasis, topically applies to his fingernails, toenails, cuticles and between the nail bed and fingernails and toenails, the emulsion of Example 3, twice a day. After 7 days of application, the subject's fingernails and toenails begin thinning and growing normally.

### Example 7

### 0.05% w/v cyclosporine emulsions vs. vehicle in a multi-patient study:

The purpose of this study was to assess the safety and efficacy of a cyclosporine emulsion versus emulsion alone both for the treatment of brittle nails and for the enhancement of normal nail growth and texture.

This was a single center, investigator-blinded (investigator-masked), head-to-head comparison of 0.05% cyclosporine emulsion Restasis® and its vehicle, Refresh® Dry Eye Therapy (which contains no cyclosporine) in 24 patients for the treatment of brittle nails and for the enhancement of normal nail growth and texture.

Three target fingernails were identified in each subject. Two of the target nails were the two nails that have the most severe signs of brittle nail syndrome. The third target nail was a normal nail or, in the absence of a normal nail, the patient's second-healthiest-appearing nail. The patient's most normal-appearing nail was untreated so that growth in the treated nails can be compared to growth in an untreated normal nail, this was the fourth target nail. Study medication was applied to target nails (the subject may also apply to all nails if they wishes, except one nail which was untreated in order to determine the subject's baseline rate of fingernail growth) for 24 weeks. The target nails were assessed at each visit. Patients were assessed at office visits every four weeks. There was a 12-week follow-up period after cessation of treatment.

The hypothesis is that daily application of Restasis® and or emulsion alone to nails and nail beds in patients with brittle nails will improve nail texture and enhance nail growth in both affected and unaffected nails. This 36-week study was conducted at one center. Adult male and female patients meeting the inclusion and exclusion criteria were screened for eligibility. The principal investigator or his/her designee obtained informed consent from all patients before any protocol-specific procedures were performed.

The primary endpoint was the proportion of patients achieving an improvement in the Physician's Global Assessment (PGA), which is a measure of the severity of brittle nail signs and symptoms in two target nails. The target nails were selected as the two fingernails that have the most severe trachyonychia (roughness), lamellar onychoschizia (peeling), or longitudinal cracking/splitting (raggedness). In addition, one normal nail was chosen as the third target nail to evaluate the effect of treatment on normal nail growth and texture. If the patient did not have a normal nail, the third target nail was the patient's second-healthiest-appearing nail. Since nail growth varies considerably among individuals, nail growth and texture of the target normal nail was compared to one untreated nail on the same hand as the target normal nail. The untreated nail was the most normal appearing nail on the same hand as the target normal nail and was the fourth target nail.

Secondary outcomes included improvement in quality of life as measured by the patient satisfaction questionnaire, difference in rate of growth of the target normal nail compared to the normal untreated nail, and the Physician's Global Improvement Assessment (PGIA) of the four target nails, which reflects the comparison of photographs taken at baseline to those taken during the study.

An analysis of the data as of December 22, 2009 by Allergan showed the following results. Nail growth in the cyclosporine group was 7.2 mm compared with 6.6 mm in the untreated nail for a 0.64 mm increase. Nail growth in the emulsion group was 5.8 mm compared to 5.7 mm in the untreated nail for a 0.17 mm increase. Cyclosporine increased nail growth 0.5 mm more than emulsion. All other measures improved nails from baseline in both the cyclosporine and emulsion groups.

For nail breakage in the primary target nail on a four point scale of 0= none, 1= mild, 2= moderate, and 3= severe. Nail breakage in the cyclosporine group was 1.55 at baseline compared with 0.91 at follow-up for a 0.64 improvement. Nail breakage in the emulsion group was 1.08 at baseline compared to 0.67 at follow-up in the emulsion nail for a 0.42 improvement. Cyclosporine improved nail breakage 0.22 points more than emulsion.

For physician global assessment of improvement for the normal nail based on texture, luster, general appearance change from baseline on a five point scale of 1= Much Better, 2= Slightly Better, 3= No Change, 4= Slightly Worse, and 5= Much Worse. Physician global improvement assessment in the cyclosporine group was 2.36 and in the emulsion group was 2.92 at follow-up. Cyclosporine improved normal nails 0.55 points more than emulsion.

## Claims

1. A pharmaceutical composition for therapeutic use in accelerating nail growth or treating brittle nails in a human or animal, the composition comprising:
a composition selected from the group consisting of an emulsion, solution, gel, paste, dispersion or suspension comprising cyclosporine.

2. A pharmaceutical composition for use according to Claim 1, wherein the cyclosporine is cyclosporine A.

3. A pharmaceutical composition for use according to Claim 1, wherein the cyclosporine is a cyclosporine A derivative or segment.

4. A pharmaceutical composition for use according to Claim 2, wherein the composition is an emulsion.

5. A pharmaceutical composition for use according to Claim 2, wherein the cyclosporine is cyclosporine A and is present in an emulsion in the amount of 0.05% w/v.

6. A pharmaceutical composition for use according to Claim 5, wherein the emulsion also contains glyerin, castor oil, polysorbate 80 and carbomer 1342.

7. A pharmaceutical composition for use according to Claim 2, wherein the cyclosporine is cyclosporine A and is present in a solution in the amount of 0.01-0.0125% w/v.

8. A pharmaceutical composition for use according to Claim 1, wherein the cyclosporine is present in the amount of 0.001-50% w/v of the composition.

9. A method for accelerating nail growth in a human or animal, the method comprising:
applying a composition selected from the group consisting of an emulsion, solution, gel, paste, dispersion or suspension comprising cyclosporine;
wherein the emulsion, solution, gel, paste, dispersion or suspension comprising cyclosporine is applied topically to the nails, cuticles or nail matrix of the human or animal at least once a day;
provided that the method is not a therapeutic method.

10. A method according to Claim 9, wherein the cyclosporine is cyclosporine A.

11. A method according to Claim 9, wherein the cyclosporine is a cyclosporine A derivative or segment.

12. A method according to Claim 10, wherein the composition is an emulsion.

13. A method according to Claim 12, wherein the cyclosporine is cyclosporine A and is present in emulsion in the amount of 0.05% w/v.

14. A method according to Claim 13, wherein the emulsion also contains glycerin, castor oil, polysorbate 80 and carbomer 1342.

15. A method according to Claim 12, wherein the cyclosporine is cyclosporine A and is present in a solution in the amount 0.01-0.125% w/v.

16. A method according to Claim 9, wherein the cyclosporine is present in the amount of 0.001-50% w/v of the composition.

## Patentansprüche

1. Pharmazeutische Zusammensetzung zur therapeutischen Verwendung zur Beschleunigung des Nagelwachstums oder zur Behandlung von brüchigen Nägeln bei Mensch und Tier, wobei die Zusammensetzung umfasst:
eine Zusammensetzung, ausgewählt aus der Gruppe bestehend aus einer Emulsion, einer Lösung, einem Gel, einer Paste, einer Dispersion oder einer Suspension, die Cyclosporin umfasst.

2. Pharmazeutische Zusammensetzung zur Verwendung gemäss Anspruch 1, wobei das Cyclosporin Cyclosporin A ist.

3. Pharmazeutische Zusammensetzung zur Verwendung gemäss Anspruch 1, wobei das Cyclosporin ein Cyclosporin A-Derivat oder -Segment ist.

4. Pharmazeutische Zusammensetzung zur Verwendung gemäss Anspruch 2, wobei die Zusammensetzung eine Emulsion ist.

5. Pharmazeutische Zusammensetzung zur Verwendung gemäss Anspruch 2, wobei das Cyclosporin Cyclosporin A ist und in einer Emulsion in einer Menge von 0,05 % Gew./Vol. vorhanden ist.

6. Pharmazeutische Zusammensetzung zur Verwendung gemäss Anspruch 5, wobei die Emulsion auch Glycerin, Rizinusöl, Polysorbat 80 und Carbomer 1342 enthält.

7. Pharmazeutische Zusammensetzung zur Verwendung gemäss Anspruch 2, wobei das Cyclosporin Cyclosporin A ist und in einer Lösung in einer Menge von 0,01 bis 0,0125 % Gew./Vol. vorhanden ist.

8. Pharmazeutische Zusammensetzung zur Verwendung gemäss Anspruch 1, wobei das Cyclosporin in einer Menge von 0,001 bis 50 % Gew./Vol. in der Zusammensetzung vorhanden ist.

9. Verfahren zur Beschleunigung des Nagelwachstums bei Mensch und Tier, wobei das Verfahren umfasst:
Auftragen einer Zusammensetzung, ausgewählt aus der Gruppe bestehend aus einer Emulsion, einer Lösung, einem Gel, einer Paste, einer Dispersion oder einer Suspension, die Cyclosporin umfasst;
wobei die/das Emulsion, Lösung, Gel, Paste, Dispersion oder Suspension, die Cyclosporin umfasst, auf die Nägel, die Nagelhaut oder die Nagelmatrix des Menschen oder Tieres mindestens einmal pro Tag topisch aufgetragen wird;
vorausgesetzt, dass das Verfahren kein therapeutisches Verfahren ist.

10. Verfahren gemäss Anspruch 9, wobei das Cyclosporin Cyclosporin A ist.

11. Verfahren gemäss Anspruch 9, wobei das Cyclosporin ein Cyclosporin A-Derivat oder -Segment ist.

12. Verfahren gemäss Anspruch 10, wobei die Zusammensetzung eine Emulsion ist.

13. Verfahren gemäss Anspruch 12, wobei das Cyclosporin Cyclosporin A ist und in der Emulsion in einer Menge von 0,05 % Gew./Vol. vorhanden ist.

14. Verfahren gemäss Anspruch 13, wobei die Emulsion auch Glycerin, Rizinusöl, Polysorbat 80 und Carbomer 1342 enthält.

15. Verfahren gemäss Anspruch 12, wobei das Cyclosporin Cyclosporin A ist und in einer Lösung in einer Menge von 0,01 bis 0,0125 % Gew./Vol. vorhanden ist.

16. Verfahren gemäss Anspruch 9, wobei das Cyclosporin in einer Menge von 0,001 bis 50 % Gew./Vol. in der Zusammensetzung vorhanden ist.

## Revendications

1. Composition pharmaceutique pour une utilisation thérapeutique destinée à accélérer la croissance des ongles ou à traiter les ongles cassants chez un humain ou un animal, la composition comprenant :
une composition choisie dans le groupe constitué d'une émulsion, d'une solution, d'un gel, d'une pâte, d'une dispersion ou d'une suspension contenant une cyclosporine.

2. Composition pharmaceutique pour une utilisation selon la revendication 1, dans laquelle la cyclosporine est la cyclosporine A.

3. Composition pharmaceutique pour une utilisation selon la revendication 1, dans laquelle la cyclosporine est un dérivé ou un segment de la cyclosporine A.

4. Composition pharmaceutique pour une utilisation selon la revendication 2, dans laquelle la composition est une émulsion.

5. Composition pharmaceutique pour une utilisation selon la revendication 2, dans laquelle la cyclosporine est la cyclosporine A et est présente dans une émulsion en une quantité de 0,05 % en poids/volume.

6. Composition pharmaceutique pour une utilisation selon la revendication 5, dans laquelle l'émulsion contient également de la glycérine, de l'huile de ricin, du polysorbate 80 et du carbomère 1342.

7. Composition pharmaceutique pour une utilisation selon la revendication 2, dans laquelle la cyclosporine est la cyclosporine A et est présente dans une solution en une quantité de 0,01 % à 0,0125 % en poids/volume.

8. Composition pharmaceutique pour une utilisation selon la revendication 1, dans laquelle la cyclosporine est présente en une quantité de 0,001 % à 50 % en poids/volume de la composition.

9. Procédé permettant d'accélérer la croissance des ongles chez un humain ou un animal, le procédé comprenant :
l'application d'une composition choisie dans le groupe constitué d'une émulsion, d'une solution, d'un gel, d'une pâte, d'une dispersion ou d'une suspension contenant une cyclosporine ;
dans lequel l'émulsion, la solution, le gel, la pâte, la dispersion ou la suspension contenant une cyclosporine est appliqué par voie topique sur les ongles, les cuticules ou la matrice unguéale de l'humain ou de l'animal au moins une fois par jour ;
à condition que le procédé ne soit pas un procédé thérapeutique.

10. Procédé selon la revendication 9, dans lequel la cyclosporine est la cyclosporine A.

11. Procédé selon la revendication 9, dans lequel la cyclosporine est un dérivé ou un segment de la cyclosporine A.

12. Procédé selon la revendication 10, dans lequel la composition est une émulsion.

13. Procédé selon la revendication 12, dans lequel la cyclosporine est la cyclosporine A et est présente dans une émulsion en une quantité de 0,05 % en poids/volume.

14. Procédé selon la revendication 13, dans lequel l'émulsion contient également de la glycérine, de l'huile de ricin, du polysorbate 80 et du carbomère 1342.

15. Procédé selon la revendication 12, dans lequel la cyclosporine est la cyclosporine A et est présente dans une solution en une quantité de 0,01 % à 0,125 % en poids/volume.

16. Procédé selon la revendication 9, dans lequel la cyclosporine est présente en une quantité de 0,001 % à 50 % en poids/volume de la composition.
